# EUROPEAN PATENT APPLICATION

(11) **EP 3 050 570 A1**
(43) Date of publication of application: **03.08.2016**
(21) Application number: 15153391.6
(22) Date of filing: 31.01.2015
(51) Int. Cl.: A61K 38/19, A61K 47/48, A61P 25/28, A61P 25/00, A61P 25/16

(54) **Pharmaceutical composition consisting of a combination of G-CSF with GM-CSF**

(71) Applicant: Neurovision Pharma GmbH, 82031 Grünwald (DE)
(72) Inventor: The designation of the inventor has not yet been filed
(74) Representative: Arth, Hans-Lothar

(57) **Abstract**

The present invention is directed to a combination of hematopoietic growth factors for use in the prophylaxis and/or treatment of neurological diseases selected from diseases associated with ischemia or hypoxia mechanisms, neurodegenerative diseases and neurological and psychiatric diseases associated with neural cell death, wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF.

## Description

The present invention is directed to a pharmaceutical composition consisting of a combination of G-CSF with GM-CSF and/or functional active variants of G-CSF having at least 90 % sequence identity to G-CSF in combination with GM-CSF and/or conjugated G-CSF in combination with GM-CSF and/or functional active variants of GM-CSF having at least 90 % sequence identity to GM-CSF in combination with G-CSF and/or conjugated GM-CSF in combination with G-CSF and/or functional active variants of GM-CSF having at least 90 % sequence identity to GM-CSF in combination with functional active variants of G-CSF having at least 90 % sequence identity to G-CSF.

The present invention is also directed to a pharmaceutical composition consisting of a combination of G-CSF with GM-CSF and/or functional active variants of G-CSF having at least 90 % sequence identity to G-CSF in combination with GM-CSF and/or conjugated G-CSF in combination with GM-CSF and/or functional active variants of GM-CSF having at least 90 % sequence identity to GM-CSF in combination with G-CSF and/or conjugated GM-CSF in combination with G-CSF and/or functional active variants of GM-CSF having at least 90 % sequence identity to GM-CSF in combination with functional active variants of G-CSF having at least 90 % sequence identity to G-CSF for use in treatment of neurological diseases selected from diseases associated with ischemia or hypoxia mechanisms, neurodegenerative diseases, traumata, traumatic brain injury, inflammation, neuroinflammation, neurological and psychiatric disorders associated with neural cell death.

Furthermore, the present invention is directed to a pharmaceutical composition consisting of a combination of G-CSF with GM-CSF and/or functional active variants of G-CSF having at least 90 % sequence identity to G-CSF in combination with GM-CSF and/or conjugated G-CSF in combination with GM-CSF and/or functional active variants of GM-CSF having at least 90 % sequence identity to GM-CSF in combination with G-CSF and/or conjugated GM-CSF in combination with G-CSF and/or functional active variants of GM-CSF having at least 90 % sequence identity to GM-CSF in combination with functional active variants of G-CSF having at least 90 % sequence identity to G-CSF for use in prophylaxis of neurological diseases selected from diseases associated with ischemia or hypoxia mechanisms, neurodegenerative diseases and neurological and psychiatric disorders associated with neural cell death.

### Background of the invention

Growth factors are proteins that are essentially involved in regulating survival, proliferation, maturation, and outgrowth of cells. Usually it is a protein or a steroid hormone. Hematopoietic growth factors are a group of glycoproteins that are described to cause blood cells to grow and mature.

Granulocyte colony-stimulating factor (G-CSF) is a 19.6-kDa glycoprotein that belongs to the group of lineage-specific hematopoietic colony growth factors. Pharmaceutically used analogs of naturally occurring G-CSF are called filgrastim and lenograstim. G-CSF is secreted by monocytes, macrophages, endothelial cells and fibroblasts and enhances the survival and proliferation of late myeloid progenitors into neutrophil granulocyte precursors. Furthermore, it stimulates precursors to differentiate into mature neutrophils and regulates neutrophil survival by inhibition of apoptosis. Anti-apoptotic G-CSF effects in the hematopoietic system are transmitted by receptor-mediated activation of the STAT (Signal Transducer and Activator of Transcription) or PI3K/Akt pathways.

The G-CSF receptor was also found to be widely expressed in different mice brain regions that are important for neurological diseases (WO 2006/008582 A1). WO 2006/008582 A1 further shows that peripherally administered G-CSF is able to cross the blood-brain barrier and is beneficial for the treatment of amyotrophic lateral sclerosis (ALS) and Parkinson's disease in mice.

All the G-CSF neuroprotection studies performed up to present in mice employed G-CSF dosing regimens higher than clinically employed dose ranges for established indications, such as treatment of chemotherapy-induced neutropenia. The corresponding studies in humans did not prove efficient in slowing down disease deterioration (Amyotr. Later. Scler. 2010, 11, 187). It is well known that due to renal clearance and degradation mediated by cells expressing the G-CSF receptor, the serum half-life of G-CSF is very short (pharmacokinetic half-time of 3-4 hours).

Side-effects of G-CSF in more than 10 in every 100 people are headaches, loss of appetite, redness and irritation at the injection site, feeling or being sick, bone pain happens in up to 4 out of 10 people (40%), diarrhea, constipation, liver changes. Furthermore, some people may have one or more of the following side effects: a skin rash, high temperature (fever), weakness, indigestion, a sore throat, tiredness (fatigue), an enlarged spleen and/or fluid build-up especially in the ankles.

EP 2049566 B1 provides G-CSF site-specific mono-conjugate derivatives, analogues and/or derivatives thereof having a glutamine residue in a position ranging from 132 to 137 of the native human 174-aminoacid G-CSF polypeptide chain sequence covalently linked to a non-immunogenic hydrophilic polymer. G-CSF and said G-CSF site-specific mono-conjugates are useful for the treatment and/or prevention of neutropenia and/or for mobilizing mammalian peripheral blood hematopoietic progenitor cells.

Granulocyte-macrophage colony-stimulating factor (GM-CSF), also known as colony stimulating factor 2 (CSF2), is a protein secreted by cells of the immune system, endothelial cells and fibroblasts. GM-CSF controls the production, differentiation, and function of granulocytes and macrophages. It is used in myeloid reconstitution following bone marrow transplant and following transplantation of autologous peripheral blood progenitor cells as well as following induction chemotherapy in older adults with acute myelogenous leukemia. The pharmaceutical analogs of naturally occurring GM-CSF are called sargramostim and molgramostim.

Adverse effects of GM-CSF occur in 20-30% of patients and usually comprise fever, myalgia, malaise, rash and injection site reaction. Early trials using very high doses of GM-CSF were associated with marked adverse effects, which in rare cases proved severe (pericarditis and thrombosis). Similarly, a so-called "first-dose reaction", defined as a syndrome of hypoxia and hypotension after the initial but not subsequent doses of GM-CSF, was observed in certain predisposed patients following doses above 10 micrograms/kg/day. Subsequent trials have established that intravenous bolus or short infusions of GM-CSF are more likely to promote adverse effects. Certain patient groups, for example those with myelodysplastic syndrome, acute myeloid leukaemia, inflammatory disease, autoimmune thrombocytopenia or malfunctional immunological responsiveness, require careful clinical monitoring in order to avoid potential complications following the administration of GM-CSF.

The inventors of WO 2004 1058287 A1 disclose that hematopoietic factors such as GM-CSF and G-CSF are sufficient to treat neurological condition. Nevertheless WO 2004 1058287 A1 does not disclose that these factors are also suitable for a prophylaxis of neurological diseases and in particular for the prophylaxis of neurodegenerative diseases. Furthermore, despite important progress in understanding pathophysiology of neurodegenerative diseases and in experimental treatment of neurodegenerative diseases, no therapeutic strategy has today proven its capability to prevent or delay onset of neurological diseases and in particular for the prophylaxis of neurodegenerative diseases.

Therefore, it is surprising that the inventors of the present invention could show in *in vivo* and *in vitro* experiments that administration of a combination of hematopoietic growth factors GM-CSF and G-CSF is suitable for prophylaxis and/or treatment of neurological diseases and in particular for the prophylaxis and/or treatment of neurodegenerative diseases, in particular in the treatment of Huntington's disease. Furthermore, it was surprisingly found that G-CSF/GM-CSF and a combined treatment of G-CSF + GM-CSF in different ratios have synergistic effects on neuronal as well as on hematopoietic progenitor cells. Further surprising was that side effects of both cytokines are used therapeutically, as e.g. induction of adequate thrombocyte values (GM-CSF), or induction of adequate monocytes (G-CSF, GM-CSF). This leads to a clear reduction of unwanted side effects (granulocytosis) and to an optimization of wanted cellular effects. Also, it was surprisingly shown that G-CSF, GM-CSF or the combination of both are able to prevent or delay the establishment of a Huntington's disease phenotype in the R6/2 mouse model and that hematopoietic growth factors GM-CSF and G-CSF can act as a medication for Huntington's disease.

It is the objective of the present invention to provide compound combinations suitable for the prophylaxis and/or treatment of neurological diseases and in particular for the prophylaxis and/or treatment of neurodegenerative diseases. Another objective of the present invention was the provision of compound combinations with reduced side effects compared to the side-effects of G-CSF and GM-CSF cause when individually applied.

The objective of the present invention is solved by the teaching of the independent claims. Further advantageous features, aspects and details of the invention are evident from the dependent claims, the description, the figures, and the examples of the present application.

### Description of the invention

Hence, the present invention is directed a combination of hematopoietic growth factors for use in the prophylaxis and/or treatment of a neurological disease selected from diseases associated with ischemia or hypoxia mechanisms, neurodegenerative diseases, neurological disorders associated with neural cell death and psychiatric disorders associated with neural cell death, wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF.

It was surprisingly found that the use of a combination of G-CSF and GM-CSF according to the present invention causes less side-effects than the use of G-CSF and/or GM-CSF alone.

The invention refers further to a method of preventing and/or treating a neurological disease selected from diseases associated with ischemia or hypoxia mechanisms, neurodegenerative diseases, traumata, traumatic brain injury, inflammation, neuroinflammation, neurological and psychiatric disorders associated with neural cell death, diseases associated with ischemia or hypoxia mechanisms, neurodegenerative diseases, traumata, traumatic brain injury, inflammation, neuroinflammation, neurological and psychiatric disorders associated with neural cell death in a mammal, comprising administering to the mammal a combination of hematopoietic growth factors, wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF in an amount sufficient for prophylaxis and/or treatment of the neurological disease, traumata and inflammation.

In other words the present invention is directed to a combination of hematopoietic growth factors for use in the manufacture of a medicament for the prophylaxis and/or treatment of a neurological disease selected from diseases associated with ischemia or hypoxia mechanisms, neurodegenerative diseases and neurological and psychiatric disorders associated with neural cell death, wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF.

"Prophylaxis" is understood to be any degree of inhibition or delay of the time of onset or severity of signs or symptoms of the disease or condition, including, but not limited to, the complete prevention of the outbreak of the disease or condition. Prophylactic administration is, hence, a preventive measure designed and used to prevent a disease and/or its symptoms and especially the disease from subsequent development. In other words, prophylaxis is any medication for a disease or condition that started before any signs or symptoms of the disease or condition occurred. The terms "prophylaxis" and "prevention" are used synonymously.

Nevertheless, recently more attention is being focused on risk factors for damaging the developing and mature nervous system, resulting in neurodegenerative diseases. Therefore several risk factors and parameters which affect our susceptibility to develop a number of neurodegenerative diseases are known and it is possible to identify a population being high-risk for development of a neurodegenerative disease.

Risk factors for neurodegenerative disease are: age, gender, family history, genetic variations or mutations, endocrine conditions, oxidative stress, inflammation, systolic blood pressure, body mass index, total cholesterol level, environmental factors, traumatic brain injury, physical activity and determination of functional loss of neurons. The present invention is especially directed to neurodegenerative diseases caused by genetic variations or mutations, wherein the genetic variation(s) or mutation(s) is/are detected by a genetic method in a sample of a person or potential patient in order to assess the prevalence or risk to obtain the neurodegenerative disease and to decide on the necessity of prophylactic use of the inventive combination of hematopoietic growth factors.

It seems that an accumulation of risk factors which appears to push the CNS over the edge triggers the neurodegenerative disease. Thus, preferred patients for the inventive prophylaxis are patients with a familial history of neurodegenerative disease(s), or identified as being genetically high-risk patients and having in addition other risk factors as mentioned above.

Consequently, the prophylactic use of the hematopoietic growth factor as disclosed herein is preferably combined with a determination of the prevalence or risk of a person or a potential patient to obtain or suffer from a neurological disease in the future. Therefore, the prophylactic use preferably involves the advance determination of the risk factor of the person or potential patient to be prophylactically treated.

The determination of the prevalence or risk of a person to obtain a neurological disease in the future can be done by biomarkers that are sensitive to potential neurological disease outbreak and/or activity. Types of biomarkers can be divided into diagnostic biomarkers that indicate the disease and initiate earlier therapies, prognostic biomarkers, that identify patterns of progression of the disease and finally monitoring biomarkers that identify ineffective drugs early on.

Thus another aspect of the present invention relates to the prophylactic use of the combination of hematopoietic growth factors, wherein the prophylaxis or prophylactic use involves determination of the prevalence or risk to obtain a neurological disease selected from diseases associated with ischemia or hypoxia mechanisms, neurodegenerative diseases, neurological and psychiatric disorders associated with neural cell death by detecting genetic mutations of the person indicating the prevalence or risk to obtain the neurological disease.

The determination of the prevalence or risk to obtain a neurological disease is based on the detection of diagnostic biomarkers selected from gene mutations in ANG (angiogenin, ribonuclease, RNase A family), OPTN (optineurin), SOD1 (copper/zinc ion-binding superoxide dismutase 1), TDP-43 (TAR DNA binding protein), C9orf72 with GGGGCC expanded repeats, FUS on chromosome 16, ATP1A3 (plasma-membrane Na⁺/K⁺ pump), UCHL1 (neuron-specific ubiquitin carboxy-terminal hydrolase-1), wherein the neurological disease is selected from ALS and/or, Alzheimer's disease and/or, Parkinson's disease and/or, Parkinson's dementia.

The determination of the prevalence or risk to obtain a neurological disease is based on the detection of diagnostic biomarkers selected from single nucleotide polymorphisms in FLJ10986, PLXDC2, FOXA2, CGNL1, NOX4, C6orf170, IQGAP2, CGNL1, Splicest EST, LOXHD1, ACCN1, TMCC2, FATE1, PTPRT, MAGI2, TACR2, DBF4B, ADAMTS20, NFIA, WNT9A, GARNL4, IL18RAP, TUFT1, TIAM2, DSC3, PARP8, DGKB, NELL1, ALK, ZNFN1A2, NGNL6975, BM924006, PRDM1, SLCO3A1, DA629334, IL2RB, PCF11, SOX21, GNG7, EIF5A, FBXO15, wherein the neurological disease is selected from ALS and/or, Alzheimer's disease and/or, Parkinson's disease and/or, Parkinson's dementia.

The determination of the prevalence or risk to obtain a neurological disease is based on the detection of diagnostic biomarkers selected from protein modifications, protein aggregation, and/or protein dysfunction shown by altered cytoplasmic distribution of OPTN, C9orf72 binding to trimethylated Histone H3 and H4 at lysine 9 (H3K9), 27 (H3K27), 79 (H3K79), 20 (H4K20), EAAT2 receptor dysfunction linked to SOD1 mutations, elevated markers of inflammation, e.g. CRP (C-reactive protein), interleukin-6 and macrophage chemotactic protein-1, systematic macrophage activation and alteration of macrophage surface markers, further epigenetic modifications, wherein the neurological disease is selected from ALS and/or, Alzheimer's disease and/or, Parkinson's disease and/or, Parkinson's dementia.

The determination of the prevalence or risk to obtain a neurological disease is based on the detection of diagnostic biomarkers selected from RNA mis-processing of TARDBP (TAR DNA binding protein), FUS (fused in sacroma), C9orf72 (chromosome 9 open reading frame 72), wherein the neurological disease is selected from ALS and/or, Alzheimer's disease and/or, Parkinson's disease and/or, Parkinson's dementia.

The determination of the prevalence or risk to obtain a neurological disease is based on the detection of diagnostic biomarkers selected from organelle dysfunction, shown by dysfunction of mitochondria based on SOD1 and TDP-43 mutations, general disruption of mitochondrial function and carbohydrate and lipid metabolism identified by imaging methods in particular MRI DEXA-scan or CT, wherein the neurological disease is selected from ALS and/or, Alzheimer's disease and/or, Parkinson's disease and/or, Parkinson's dementia.

The determination of the prevalence or risk to obtain a neurological disease is based on the detection of diagnostic biomarkers selected from altered mRNA levels and/or altered expression of genes, shown by down-regulated MDH1 (cytosolic malate dehydrogenase 1), down-regulation of genes encoding NDUFA4 (protein of complex 1 of the electron transport chain in the inner mitochondrial membrane), down-regulation of CYCS (protein facilitating electron transport form complex III to complex IV in the intramembrane space) represents a cytoplasmic signal for neuronal apoptosis, down-regulation of ATP5A1 (protein of the ATP synthase in the inner mitochondrial membrane), down-regulation of ANGPTL4, CHGB (granin protein), SCG5 (granin protein), PCSK1 (prohormone convertase) expression, down-regulated KCNC2 (voltage-dependent potassium ion permeability regulator of excitable membranes), down-regulation of PVALB (calcium buffer protein), DSCR1L1 (inhibitor of calcineurin), NRGN (calmodulin binding protein), PCP4 (calmodulin binding protein), up-regulation of AQP1 (urea and water transport protein), SLC14A1 (urea and water transport protein), down-regulation of microtubules, deregulation of TUBB2A (tubulin beta protein), TUBB6 (tubulin beta protein), decreased expression of NEFL (neurofilament subunit), NEF3 (neurofilament subunit), NEFH (neurofilament subunit), increased miR-34c level, up-regulated MLLT11 expression, up-regulated OLFM1, down-regulation of BEX1 (promoter of cell proliferation), down-regulation of RTN1 and RTN4 (members of the reticulon family), down-regulation of PNMA2, up-regulation of FCGBP (encoding IgG-Fc binding protein), up-regulation of CEBPD (encoding a transcription factor of cytokines and other pro-inflammatory response genes), DNA marker analysis of the D21 S58 region on chromosome 21, wherein the neurological disease is selected from ALS and/or, Alzheimer's disease and/or, Parkinson's disease and/or, Parkinson's dementia.

It is preferred to start with the prophylaxis or prophylactic use immediately after detection of the prevalence or risk to obtain the neurological disease. That means, in case the genetic test of the genetic material of a person or potential patient indicates that the person or potential patient has an increased or high risk to develop in the future a neurological disease or more preferred a neurodegenerative disease, the prophylactic use of the inventive combination of hematopoietic growth factors should be started immediately.

Consequently the present invention relates to a method for prophylaxis of a neurological disease of a patient having an increased or high risk to develop the neurological disease by determining the increased or high risk to develop the neurological disease and administering to that patient a pharmacologically effective amount of an hematopoietic growth factor which is selected from the group consisting of G-CSF, GM-CSF, functional active variants thereof having at least 90 % sequence identity to G-CSF, functional active variants thereof having at least 90 % sequence identity to GM-CSF, conjugated G-CSF, and conjugated GM-CSF. Preferred is the administration of a combination of two hematopoietic growth factors as disclosed herein.

More preferred is a method for prophylaxis of a neurodegenerative disease of a patient having an increased or high risk to develop the neurodegenerative disease by determining the increased or high risk to develop the neurodegenerative disease and administering to that patient a pharmacologically effective amount of a combination of hematopoietic growth factors wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF.

The prevalence or increased or high risk to develop a neurological disease or a neurodegenerative disease is determined by testing a sample of the person or potential patient such as a blood sample or saliva sample for genetic mutations or variations as mentioned herein.

As used herein, the term "neurological diseases" encompasses diseases with ischemic or hypoxic mechanisms, neurodegenerative diseases (genetic, paraneoplastic, autoimmune, metabolic), inflammation and traumata, neuroinflammation, traumatic brain injury, and neurological and psychiatric diseases associated with neural cell death.

The term "functional active variant" refers to peptides having at least 90 % sequence identity to G-CSF or GM-CSF wherein said peptide is a bioactive Granulocyte Colony Stimulating Factor peptide or respectively, a bioactive Granulocyte Macrophage Colony Stimulating Factor peptide, hence, being a cytokine that functions as a white blood cell growth factor.

Neurodegenerative diseases are a group of usually slowly progressive, hereditary or sporadic diseases of the nervous system. The main feature is the increasing loss of nerve cells, which results in various neurological symptoms, including frequent dementia and movement disorders. The disease can occur at different ages, run diffuse or generalized and elicit characteristic histological pattern of damage.

Diseases with ischemic or hypoxic mechanisms can be classified into general diseases and cerebral ischemia. Examples of such general diseases involving ischemic or hypoxic mechanisms include myocardial infarction, cardiac insufficiency, cardiac failure, congestive heart failure, myocarditis, pericarditis, perimyocarditis, coronary heart disease (stenosis of coronary arteries), angina pectoris, congenital heart disease, shock, ischemia of extremities, stenosis of renal arteries, diabetic retinopathy, thrombosis associated with malaria, artificial heart valves, anemias, hypersplenic syndrome, emphysema, lung fibrosis, and pulmonary edema. Examples of cerebral ischemia disease include stroke (as well as hemorrhagic stroke), cerebral microangiopathy (small vessel disease), intrapartal cerebral ischemia, cerebral ischemia during/after cardiac arrest or resuscitation, cerebral ischemia due to intraoperative problems, cerebral ischemia during carotid surgery, chronic cerebral ischemia due to stenosis of blood-supplying arteries to the brain, sinus thrombosis or thrombosis of cerebral veins, cerebral vessel malformations, and diabetic retinopathy.

Examples of neurodegenerative diseases include all genetic and spontaneous forms of amyotrophic lateral sclerosis (ALS), all types of dementias such as Alzheimer's disease, frontotemporal dementia and Pick's disease, Parkinson's disease and Parkinson-like diseases, Huntington's disease, spinal muscular-atrophy, progressive supranuclear palsy (Steele-Richardson-Olszewski syndrome), Wilson's disease, multi-system atrophy (MSA-P, MSA-C), striatonigral degeneration, Shy-Drager syndrome, spinocerebellar ataxia (SCA), glaucoma, Lewy-body disease, Hallervorden-Spatz disease, torsion dystonia, hereditary sensorimotor neuropathies (HMSN), Gerstmann-Straussler-Schanker disease, Creutzfeld-Jakob-disease, Machado-Joseph disease, Friedreich ataxia, non-Friedreich ataxias, Gilles de la Tourette syndrome, familial tremors, olivopontocerebellar degenerations, paraneoplastic cerebral / cerebellar syndromes, hereditary spastic paraplegias, hereditary optic neuropathy (Leber optic atrophy), degenerative retinal diseases, retinitis pigmentosa, dry and humid macular degeneration, Stargardt disease, and Kearns- Sayre syndrome.

According to a preferred embodiment of the invention, the neurodegenerative disease is Parkinson's disease, in particular Parkinson's dementia, amyotrophic lateral sclerosis (ALS) and Alzheimer's disease.

"Treatment" is understood to be any degree of inhibition or delay of the onset or severity of signs or symptoms of the disease or condition, including, but not limited to, the complete cure of the disease or condition. In other words, treatment is any medication for a disease or condition that started after any signs or symptoms of the disease or condition occurred. Hence, treatment is designed and used to medicate a disease and/or its symptoms and especially prevent the disease from subsequent development.

Examples of neurological and psychiatric diseases associated with neural cell death include septic shock, intracerebral bleeding, subarachnoidal hemorrhage, multiinfarct dementia, inflammatory diseases (such as vasculitis, multiple sclerosis, and Guillain-Barre-syndrome), neurotrauma (such as spinal cord trauma, and brain trauma), peripheral neuropathies, polyneuropathies, epilepsies, schizophrenia, depression, metabolic encephalopathies, and infections of the central nervous system (viral, bacterial, fungal) such as AIDS-associated dementia.

Conjugation is routinely achieved by incubation of a reactive or functionalized polymer, such as a functionalized PEG, with the target protein. Physical and chemical changes by conjugation mostly increase systemic retention of the therapeutic agent. For proteins, typical amino acids suitable for conjugation include lysine, cysteine, histidine, arginine, aspartic acid, glutamic acid, serine, threonine, tyrosine. The N-terminal amino group and the C-terminal carboxylic acid can also be used as a site specific site by conjugation with aldehyde functional polymers.

One preferred embodiment of the present invention refers to a combination of hematopoietic growth factors wherein the conjugated G-CSF consists of a non-immunogenic hydrophilic polymer covalently linked to G-CSF or wherein the conjugated GM-CSF consists of a non-immunogenic hydrophilic polymer covalently linked to GM-CSF.

It is preferred that the non-immunogenic hydrophilic polymer is selected from the group comprising or consisting of linear or branched polyethylene glycols, polyoxypropylenes, polyoxyethylene-polyoxypropylene block copolymers, polyvinylpyrrolidones, polyacyloylmorpholines, polysaccharides, and aminocarbamyl polyethylene glycols. Even more preferred the non-immunogenic hydrophilic polymer is selected from linear or branched monomethoxy-polyethylene glycol amine and aminocarbamyl polyethylene glycol having a molecular weight from 5kDa to 40kDa and more preferably from 15KDa to 25KDa. Especially preferred is a linear monomethoxy-polyethylene glycol amine or an aminocarbamyl polyethylene glycol of molecular weight of 20KDa.

Thus, a preferred non-immunogenic hydrophilic carrier is a monomethoxy-polyethylene glycol amine of the following general formula: 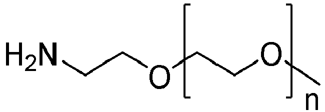 wherein n ranges from 112 to 907, preferably from 339 to 566, and more preferably is 453.

Another preferred non-immunogenic hydrophilic carrier is a O-[methyl-poly(ethylene glycol)]-*N*-[2-(3-aminopropoxy)ethoxy]ethylcarbamate of the following general formula: 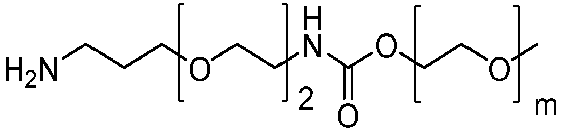 wherein m ranges from 109 to 904, preferably from 336 to 563, and is more preferably 450.

One preferred embodiment of the present invention refers to a site-specific G-CSF mono-conjugate for use in the prophylaxis of a neurological disease selected from diseases associated with ischemia or hypoxia mechanisms, neurodegenerative diseases and neurological and psychiatric disorders associated with neural cell death, wherein the site-specific G-CSF mono-conjugate consists of a non-immunogenic hydrophilic polymer covalently linked to a glutamine residue in the position 134 of the native human 174-aminoacid G-CSF polypeptide chain sequence or 135 of the 175-aminoacid Met-G-CSF polypeptide chain sequence.

The term "non-immunogenic" refers to the hydrophilic polymers that are covalently linked to an amino acid of G-CSF or GM-CSF and are in particular covalently linked to the glutamine residue in the position 134 of the native human 174-aminoacid G-CSF polypeptide chain sequence or 135 of the 175-aminoacid Met-G-CSF polypeptide chain sequence. The term "non-immunogenic" implies that said hydrophilic polymers, when administered through the systemic route do not induce immune system activation, nor significantly cause specific anti hydrophilic polymer antibodies.

The term "hydrophilic polymers" has the same meaning as disclosed in EP 2049566 B1 i.e. linear or branched hydrophilic polymers that are functionalized with a primary amino group so that they can be site-specific linked *via* an amide bond to the acyl moiety of the single glutamine residue adjacent to the threonine residue that is glycosylated in native G-CSF. The site-specific covalent linkage is achieved by a transglutaminase enzyme catalyzed reaction.

The native human 174-aminoacid G-CSF polypeptide chain sequence is the following (the glutamine residue that is covalently bound to the non-immunogenic hydrophilic polymer is bolded) (Seq ID No. 1):

Thr Pro Leu Gly Pro Ala Ser Ser Leu Pro
Gln Ser Phe Leu Leu Lys Cys Leu Glu Gln

Val Arg Lys Ile Gln Gly Asp Gly Ala Ala
Leu Gln Glu Lys Leu Cys Ala Thr Tyr Lys
Leu Cys His Pro Glu Glu Leu Val Leu Leu
Gly His Ser Leu Gly Ile Pro Trp Ala Pro
Leu Ser Ser Cys Pro Ser Gln Ala Leu Gln
Leu Ala Gly Cys Leu Ser Gln Leu His Ser
Gly Leu Phe Leu Tyr Gln Gly Leu Leu Gln
Ala Leu Glu Gly Ile Ser Pro Glu Leu Gly
Pro Thr Leu Asp Thr Leu Gln Leu Asp Val
Ala Asp Phe Ala Thr Thr Ile Trp Gln Gln
Met Glu Glu Leu Gly Met Ala Pro Ala Leu
Gln Pro Thr Gln Gly Ala Met Pro Ala Phe
Ala Ser Ala Phe Gln Arg Arg Ala Gly Gly
Val Leu Val Ala Ser His Leu Gln Ser Phe
Leu Glu Val Ser Tyr Arg Val Leu Arg His
Leu Ala Gln Pro

The 175-aminoacid Met-G-CSF polypeptide chain sequence is the following (the glutamine residue that is covalently bound to the non-immunogenic hydrophilic polymer is bolded) (Seq ID No. 2):

Met Thr Pro Leu Gly Pro Ala Ser Ser Leu
Pro Gln Ser Phe Leu Leu Lys Cys Leu Glu
Gln Val Arg Lys Ile Gln Gly Asp Gly Ala
Ala Leu Gln Glu Lys Leu Cys Ala Thr Tyr
Lys Leu Cys His Pro Glu Glu Leu Val Leu
Leu Gly His Ser Leu Gly Ile Pro Trp Ala
Pro Leu Ser Ser Cys Pro Ser Gln Ala Leu
Gln Leu Ala Gly Cys Leu Ser Gln Leu His
Ser Gly Leu Phe Leu Tyr Gln Gly Leu Leu
Gln Ala Leu Glu Gly Ile Ser Pro Glu Leu
Gly Pro Thr Leu Asp Thr Leu Gln Leu Asp
Val Ala Asp Phe Ala Thr Thr Ile Trp Gln
Gln Met Glu Glu Leu Gly Met Ala Pro Ala
Leu Gln Pro Thr Gln Gly Ala Met Pro Ala
Phe Ala Ser Ala Phe Gln Arg Arg Ala Gly
Gly Val Leu Val Ala Ser His Leu Gln Ser
Phe Leu Glu Val Ser Tyr Arg Val Leu Arg
His Leu Ala Gln Pro

The non-immunogenic hydrophilic polymer used for accessing the site-specific G-CSF mono-conjugates of the present invention is preferably selected from the group comprising or consisting of linear or branched polyethylene glycols, polyoxypropylenes, polyoxyethylene-polyoxypropylene block copolymers, polyvinylpyrrolidones, polyacyloylmorpholines, polysaccharides and aminocarbamyl polyethylene glycols. More preferably, the non-immunogenic hydrophilic polymer is selected from linear or branched monomethoxy-polyethylene glycol amine and aminocarbamyl polyethylene glycol having a molecular weight from 5kDa to 40kDa and more preferably from 15KDa to 25KDa. Especially preferred is a linear monomethoxy-polyethylene glycol amine or an aminocarbamyl polyethylene glycol of molecular weight of 20KDa.

Thereby the non-immunogenic hydrophilic carrier is conjugated to the native human 174-aminoacid G-CSF or to the 175-aminoacid Met-G-CSF following the conjugation procedure disclosed by EP 2049566 B1. Said conjugation procedure employs a microbial transglutaminase (for example: *Streptoverticillium mobaraense* MTG enzyme) and includes the following steps:
- dissolution of native human 174-aminoacid G-CSF or 175-aminoacid Met-G-CSF in a buffer solution with a pH ranging from 6 to 8, preferably of 7.4;
- addition of the non-immunogenic hydrophilic polymer, displaying at least one primary amino function (for example: monomethoxy-polyethylene glycol amine, *O-*[methyl-poly(ethyleneglycol)]-*N*-[2-(3-aminopropoxy)ethoxy]ethylcarbamate);
- addition of MTG and reaction for 1 - 24 hours at a temperature ranging between 15°C and 35°C, preferably 25°C;
- purification of site-specific mono-conjugated G-CSF, preferably by column chromatography.

Surprisingly, the above mentioned site-specific G-CSF mono-conjugates proved particularly efficient for the prophylaxis and/or treatment of neurological diseases.

Another aspect of the present invention refers to a combination of hematopoietic growth factors for use in the prophylaxis and/or treatment of a neurological disease selected from diseases associated with ischemia or hypoxia mechanisms, neurodegenerative diseases and neurological and psychiatric disorders associated with neural cell death, wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF and wherein the use comprises the intrathecal and/or intracerebroventricular administration of the combination of hematopoietic growth factors.

Another embodiment of the present invention is directed to the combination of site-specific G-CSF and GM-CSF mono-conjugates for use in the prophylaxis and/or treatment of neurological diseases, the pharmaceutical composition comprising the site-specific G-CSF mono-conjugate and GM-CSF, and the pharmaceutical composition comprising the site-specific G-CSF mono-conjugate and GM-CSF covalently linked to a non-immunogenic hydrophilic polymer contained in the reservoir of a device for intrathecal / intracerebroventricular administration.

Another embodiment of the present invention is directed to the pharmaceutical composition comprising the site-specific GM-CSF mono-conjugate and G-CSF, and the pharmaceutical composition comprising the site-specific GM-CSF mono-conjugate and G-CSF covalently linked to a non-immunogenic hydrophilic polymer contained in the reservoir of a device for intrathecal / intracerebroventricular administration.

The term "intrathecal administration" refers to an administration into the intrathecal space or subarachnoid space defined by pia mater and arachnoid, where the drug comes into contact with the cerebrospinal fluid that transports the drug to the ventricles. The term "intracerebroventricular administration" refers to an administration into the ventricular system of the brain. Both methods bypass the blood-brain barrier which limits drug distribution into the brain, allowing high drug concentrations to enter the central compartment. Administration of drugs directly into the ventricles of the brain must be done consideration of factors affecting the efficacy and safety of this route of administration. These factors include the osmolarity, pH, volume, and presence of preservatives and diluents of the pharmaceutical composition being administered.

Another aspect of the present invention refers to a combination of hematopoietic growth factors for use in the prophylaxis and/or treatment of a neurological disease selected from diseases associated with ischemia or hypoxia mechanisms, neurodegenerative diseases and neurological and psychiatric diseases associated with neural cell death, wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF and wherein the use comprises administration using a viral vector, a virus-like particle or a liposome.

A viral vector, virus-like particles or a liposome can be used as a vehicle for administration of pharmaceutical drugs. These pharmaceutical carriers are used to deliver drugs more efficiently past the lipid bilayer of cell membranes. A liposome is an artificially-prepared spherical vesicle composed of a lamellar phase lipid bilayer. The lipid bilayer can fuse with other bilayers such as the cell membrane, thus delivering the liposome contents.

Viruses have evolved specialized molecular mechanisms to efficiently transport their genomes inside the cells they infect. One can take advantage of these mechanisms by using viral vectors or virus-like particles to deliver therapeutic agents into tissue and cells. Numerous different viral vector systems have been developed for *in vitro, ex vivo* and *in vivo* gene transfer, which were mainly derived from murine and human DNA- and RNA-viruses. To create recombinant viral vectors viruses are manipulated to be still capable of infecting their target cells and delivering their load, but fail to harm the host cell (such as lysis). Virus-like particles (VLPs) are multiprotein structures that mimic the organization and conformation of authentic native viruses, in particular their capsids with or without an "envelope" (lipids derived from the host cell), but lack the viral genome. The expression of viral structural proteins, such as capsomers, can result in the self-assembly of virus like particles (VLPs).

A beneficial synergistic effect was observed when G-CSF was administered to patients having an increased risk to suffer from neurological diseases in combination with GM-CSF or GM-CSF covalently linked to a non-immunogenic hydrophilic polymer. Therefore, the present invention relates further to a combination of hematopoietic growth factors for use in the prophylaxis and/or treatment of a neurological disease selected from diseases associated with ischemia or hypoxia mechanisms, neurodegenerative diseases and neurological and psychiatric diseases associated with neural cell death, wherein the use comprises administration of a combination of two hematopoietic growth factors selected from G-CSF together with GM-CSF, G-CSF together with a conjugated G-CSF and G-CSF together with a conjugated GM-CSF.

An embodiment of the present invention is a pharmaceutical composition for use in the prophylaxis and/or treatment of neurological diseases selected from diseases associated with ischemia or hypoxia mechanisms, neurodegenerative diseases as well as neurological and psychiatric diseases associated with neural cell death, wherein the pharmaceutical composition comprises a combination of hematopoietic growth factors wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF.

Another aspect of the present invention is directed to hematopoietic growth factors as disclosed herein for use in prophylaxis and/or treatment of a neurological disease, wherein the use comprises administration of a combination of two hematopoietic growth factors the first one selected from the group consisting of G-CSF, functional active variants of G-CSF having at least 90 % sequence identity to G-CSF and conjugated G-CSF, and the second one selected from the group consisting of GM-CSF, functional active variants of GM-CSF having at least 90 % sequence identity to GM-CSF and conjugated GM-CSF.

Another embodiment of the present invention is directed to a pharmaceutical composition for use in the prophylaxis and/or treatment of neurological diseases selected from diseases associated with ischemia or hypoxia mechanisms, neurodegenerative diseases as well as neurological and psychiatric diseases associated with neural cell death, wherein the pharmaceutical composition comprises a combination of hematopoietic growth factors wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF together with at least one pharmaceutically acceptable cryoprotectant, lyoprotectant, excipient and/or diluent for use in the prophylaxis and/or treatment of neurological diseases. Such pharmaceutical composition includes ready-to-use sterile solutions and sterile lyophilized powders to be reconstituted for use with an appropriate solvent.

Thus, the present invention is related to a pharmaceutical composition containing a combination of two hematopoietic growth factors in a ratio of 4 to 1 of the first hematopoietic growth factor to the second hematopoietic growth factor and the first hematopoietic growth factor is selected from the group consisting of G-CSF, functional active variants of G-CSF having at least 90 % sequence identity to G-CSF and conjugated G-CSF, and the second hematopoietic growth factor is selected from the group consisting of GM-CSF, functional active variants of GM-CSF having at least 90 % sequence identity to GM-CSF and conjugated GM-CSF together with a pharmaceutically acceptable solvent, diluent, carrier and/or adjuvant.

Furthermore, the present invention is also related to the administration of a ratio of the combination of the two hematopoietic growth factors, wherein the ratio depends on the determination of the prevalence or risk to obtain a neurological disease, wherein the prevalence or risk to obtain a neurological disease is between 80% and 100%, a pharmaceutical composition containing a combination of two hematopoietic growth factors in a ratio of 1 to 1 of the first hematopoietic growth factor to the second hematopoietic growth factor should be administered, and the first hematopoietic growth factor is selected from the group consisting of G-CSF, functional active variants of G-CSF having at least 90 % sequence identity to G-CSF and conjugated G-CSF, and the second hematopoietic growth factor is selected from the group consisting of GM-CSF, functional active variants of GM-CSF having at least 90 % sequence identity to GM-CSF and conjugated GM-CSF together with a pharmaceutically acceptable solvent, diluent, carrier and/or adjuvant.

Another aspect of the present invention is also related to the administration of a ratio of the combination of the two hematopoietic growth factors, wherein the ratio depends on the determination of the prevalence or risk to obtain a neurological disease, wherein the prevalence or risk to obtain a neurological disease is between 50% and 80%, a pharmaceutical composition containing a combination of two hematopoietic growth factors in a ratio of 2 to 1 of the first hematopoietic growth factor to the second hematopoietic growth factor should be administered, and the first hematopoietic growth factor is selected from the group consisting of G-CSF, functional active variants of G-CSF having at least 90 % sequence identity to G-CSF and conjugated G-CSF, and the second hematopoietic growth factor is selected from the group consisting of GM-CSF, functional active variants of GM-CSF having at least 90 % sequence identity to GM-CSF and conjugated GM-CSF together with a pharmaceutically acceptable solvent, diluent, carrier and/or adjuvant.

Another aspect of the present invention is also related to the administration of a ratio of the combination of the two hematopoietic growth factors, wherein the ratio depends on the determination of the prevalence or risk to obtain a neurological disease, wherein the prevalence or risk to obtain a neurological disease is between 1% and 50%, a pharmaceutical composition containing a combination of two hematopoietic growth factors in a ratio of 1 to 2 of the first hematopoietic growth factor to the second hematopoietic growth factor should be administered, and the first hematopoietic growth factor is selected from the group consisting of G-CSF, functional active variants of G-CSF having at least 90 % sequence identity to G-CSF and conjugated G-CSF, and the second hematopoietic growth factor is selected from the group consisting of GM-CSF, functional active variants of GM-CSF having at least 90 % sequence identity to GM-CSF and conjugated GM-CSF together with a pharmaceutically acceptable solvent, diluent, carrier and/or adjuvant.

The pharmaceutical compositions of the present invention are particularly suitable for parenteral administration. Preferably, the pharmaceutical compositions for use in the treatment of neurological diseases are administered subcutaneously, intramuscularly, intravenously, intraarterially, by direct injection to the brain or intrathecally or intracerebroventricularly. The intrathecal / intracerebroventricular administration is especially preferred. The injection can be performed using pre-filled syringes, devices comprising pre-filled reservoirs or similar means.

A ready-to-use sterile solution comprises for example a combination of hematopoietic growth factors wherein the concentrations of the hematopoietic growth factors are ranging from 1 to 10 mg/ml per growth factor, preferably from 5 to 10mg/ml per growth factor and an isotonic agent selected, for example, amongst sugars such as sucrose, lactose, mannitol or sorbitol. A suitable buffering agent, to control the solution pH to 4 or 5 (preferably 5), may be also included. Another optional ingredient of the formulation can be a non-ionic surfactant, such as Tween 20 or Tween 80.

Preferably, the hematopoietic growth factor administered for prophylactic use involves administration of the combination of hematopoietic growth factors wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF in a single dose in the range of 0,5µg/kg to 5 µg/kg, preferably in the range of 1 µg/kg to 3 µg/kg, and more preferably 2 µg/kg per day.

Preferably, the hematopoietic growth factor administered for treatment involves administration of the combination of hematopoietic growth factors wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF in a single dose in the range of 0,5µg/kg to 5 µg/kg, preferably in the range of 1 µg/kg to 3 µg/kg, and more preferably 2 µg/kg per day.

A sterile lyophilized powder to be reconstituted for use comprises the hematopoietic growth factor, and optionally a bulking agent (e.g. mannitol, trehalose, sorbitol, glycine) and/or a cryoprotectent (e.g. trehalose, mannitol). The solvent for reconstitution can be water for injectable compounds, with or without a buffering salt to control the pH to 4 to 5.

To facilitate the delivery to the brain, a device for intra brain or intrathecal or intracerebroventricular administration comprising a catheter and the pharmaceutical composition containing the combination of hematopoietic growth factors wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF. Such device can be an implantable one, wherein at least part of the device enters the brain. For example, the catheter of the device can be inserted into the lateral ventricle of brain, into the parenchyma tissue of brain or into the cisterna magna of brain. In intrathecal administration, the catheter can be inserted into the subarachnoid space (intrathecal space).

The device can further comprise a reservoir containing the pharmaceutical composition, which comprises the combination of hematopoietic growth factors wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF linked to a non-immunogenic hydrophilic polymer. Hence, such a device comprises a reservoir containing the pharmaceutical composition, wherein said reservoir is in fluid communication with the catheter for delivering the pharmaceutical composition to the brain or to the cerebrospinal fluid. In other words the reservoir is coupled to the catheter that is adapted to deliver the combination of hematopoietic growth factors or the pharmaceutical composition to the brain or to the cerebrospinal fluid. The reservoir containing the pharmaceutical composition can be implanted in the brain or attached to the outer side of the scalp of the patient. The reservoir is replenishable.

Additionally, the device may comprise a fluid transfer device for releasing the pharmaceutical composition contained in the reservoir. Thus, such a device comprises a reservoir containing the pharmaceutical composition, a fluid transfer device for releasing the pharmaceutical composition and a catheter for delivering the pharmaceutical composition. The fluid transfer device is in direct contact with the reservoir containing the pharmaceutical composition. The fluid transfer device can be implanted in the brain or attached to the outer side of the scalp of the patient and might be a pump or a valve. The pump can be an electrical pump, a battery operated pump, an osmotic pump, a vapor pressure-activated pump, a peristaltic pump or any combination thereof.

The present invention further provides a prefilled device for intrathecal / intracerebroventricular administration of the combination of hematopoietic growth factors wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF comprising:
- a pump;
- a catheter coupled to the reservoir and adapted to deliver the combination of hematopoietic growth factors wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF in the intrathecal / intracerebroventricular space of a patient,
- wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF, which are both contained in the reservoir of the device and deliverable through the catheter.

Thus, an embodiment of the present invention is directed to a prefilled device for intrathecal / intracerebroventricular administration of the inventive use of the combination of hematopoietic growth factors, or of the pharmaceutical composition comprising the hematopoietic growth factors, wherein the hematopoietic growth factors or the pharmaceutical composition are contained in a reservoir within the device. Said prefilled device for intrathecal / intracerebroventricular administration is implantable and the content of the reservoir can be refilled once it was emptied.

Optionally, the device may further comprise a control means adapted to determine the concentration of the hematopoietic growth factor in the cerebrospinal fluid of a patient. In function of the determined concentration values, the flow of the hematopoietic growth factor or of the pharmaceutical composition delivered by the catheter can be adjusted.

The following examples are included to demonstrate preferred embodiments of the invention. It should be appreciated by those of skill in the art that the techniques disclosed in the following examples represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

### A: Experimental design for in vitro Lipegfilgrastim (Lonquex®) / G-CSF / GM-CSF-mediated effects to enhance activation of adult neurogenesis:

Increased CSF (cerebrospinal fluid) TGF-β levels are known to be a hallmark of neurodegenerative processes resulting in reduced neurogenesis. The major goal of the following experiments was to investigate whether Lipegfilgrastim (Lonquex®), granulocyte-colony stimulating factor (G-CSF), granulocyte-macrophage colony-stimulating factor (GM-CSF) or the combination of G-CSF + GM-CSF or Lipegfilgrastim (Lonquex®) + GM-CSF is able to prevent, reactivate or compensate the impaired properties of cells, which are induced by increased levels of circulating TGF-β in a dose-dependent manner.

In the *in vitro*-experiments the main question was whether Lipegfilgrastim (Lonquex®), G-CSF, GM-CSF or a combined treatment of Lipegfilgrastim (Lonquex®) + GM-CSF / G-CSF + GM-CSF in different ratios will have synergistic effects on neuronal as well as on hematopoietic progenitor cells. Side effects of each cytokine should be used therapeutically, as e.g. induction of adequate thrombocyte values (GM-CSF), or induction of adequate monocytes (G-CSF, GM-CSF). By molecular fingerprinting potential combined or synergistic effects have been monitored and described. This leads to a clear reduction of unwanted side effects (granulocytosis) and to an optimization of wanted cellular effects.

### Experiment A1: Treatment of human neuronal progenitor cell line with Lipegfilgrastim (Lonquex®) / G-CSF / GM-CSF / Lipegfilgrastim (Lonquex®) + GM-CSF / G-CSF + GM-CSF

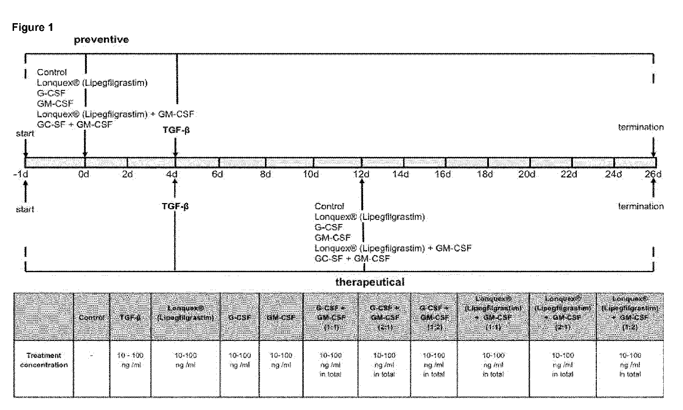

Single or combined treatment of Lipegfilgrastim (Lonquex®), G-CSF or GM-CSF may interact with neurogenesis and therefore - as a model system -ReN-CX cells (a human neuronal progenitor cell line) received Lipegfilgrastim (Lonquex®), G-CSF or GM-CSF alone or in different combination ratios of Lipegfilgrastim (Lonquex®) + GM-CSF or G-CSF + GM-CSF (1:1, 2:1, 1:2) after (therapeutical) or without (preventive) previously exposing the cells to high TGF-β-levels. Human neuronal progenitor cells have been investigated regularly (every 2 days) with regard to cell behavior: proliferation (immunhistochemistry Ki67, BrdU Assay), cell migration (scratch assay) and differentiation (immunhistochemistry (Nestin, βIII-Tubulin, Neu N)). In addition the cell supernatant was analyzed for cytokines including IL-3, II-4, IFN-γ, II-17, BDNF, Eotaxin, II-6, TNF-alpha, NFκB, M-CSF, SCF using Meso Scale Discovery assay platform (MSD, Gaithersburg, Maryland, USA). Also metabolites of this interaction were observed and analyzed by MS. From the Biomarkers a profile of interaction has been characterized, evaluating the grade and quality of interaction in neuronal cells. The timeline and sequence of administration as well as the administered concentrations are shown in figure 1.

### Experiment A2: Treatment of myeloid precursor cells of healthy controls versus ALS samples with Lipegfilgrastim (Lonquex®) / G-CSF / GM-CSF / Lipegfilgrastim (Lonquex®) + GM-CSF / G-CSF + GM-CSF

The major goal of experiment A2 was to investigate whether single treatment of Lipegfilgrastim (Lonquex®); G-CSF, GM-CSF or the combination of Lipegfilgrastim (Lonquex®) + GM-CSF, G-CSF + GM-CSF exerts preventive or therapeutic synergistic effects, leading to an expanded proliferation and differentiation of monocytes and a reduced risk of thrombocytosis and myeloid precursor cells of healthy controls versus ALS patients. Therefore, samples were treated with Lipegfilgrastim (Lonquex®), G-CSF, GM-CSF alone or with different ratios of Lipegfilgrastim (Lonquex®) + GM-CSF, G-CSF + GM-CSF (1:1, 2:1, 1:2). Every 2 days cells were analyzed for cell type discrimination (FACS Analysis), proliferation ratios (immunhistochemistry Ki67, FACS), cell migration (scratch assay) and differentiation (CFU Assay, FACS) and the experiment was finished after 26 days. In addition, cell supernatant has been investigated with regard to different cytokines like IL-3, II-4, IFN-γ, II-17, BDNF, Eotaxin, II-6, TNF-alpha, NFκB, M-CSF, SCF, using Meso Scale Discovery assay platform (MSD, Gaithersburg, Maryland, USA). Additionally, mRNA-profile of Triggering receptor expressed on myeloid cells 2 (TREM2) has been analyzed by quantitative real-time RT-PCR. This receptor is known to be expressed by myeloid cells and mutations in the coding gene are supposed to play a role in neurodegenerative diseases. Also metabolites of this interaction were observed and analyzed by mass spectrometry (MS). From the Biomarkers a profile of interaction has been characterized, evaluating the grade and quality of interaction in myeloid cells. The timeline and sequence of administration as well as the administered concentrations are shown in figure 1.

### Experiment A3: Treatment of immortalized murine microglia cells with Lipegfilgrastim (Lonquex®) / G-CSF / GM-CSF or Lipegfilgrastim (Lonquex®) + GM-CSF / G-CSF + GM-CSF

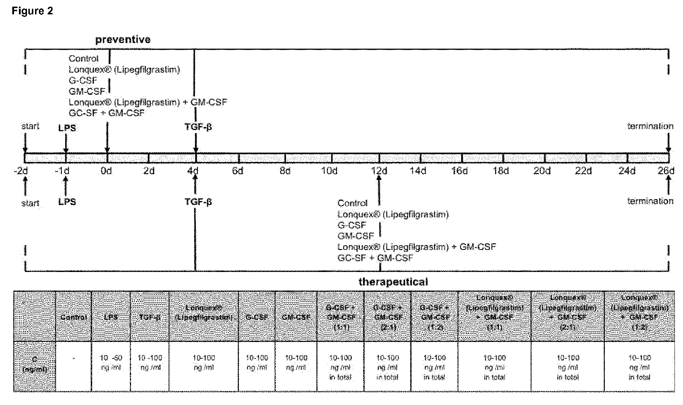

Experiment A3 showed that combination treatments with Lipegfilgrastim (Lonquex®) + GM-CSF or G-CSF and GM-CSF resulted in a reduced toxicity compared to a single drug administration, which lead to an enhanced survival of neuronal precursor cells. Lipopolysaccarid (LPS), an endotoxin, known to be a component in the outer membrane of gram-negative bacteria can lead to a strong immune response. But it can also be used to activate ramified microglia in cell culture. Following LPS-activation, BV2-cells get activated and could be distinguished by Iba-1 expression. Furthermore to examine toxicity, activated BV-2 cells were also split into two groups with one group first exposed to high levels of TGF-β and subsequently to Lipegfilgrastim (Lonquex®), G-CSF, GM-CSF or combination treatments in different ratios (1:1, 2:1, 1:2), respectively (therapeutical group). The second group was first exposed to Lipegfilgrastim (Lonquex®), G-CSF, GM-CSF or with the combination treatments in different ratios (1:1, 2:1, 1:2), respectively and subsequently to high levels of TGF-ß (prevention group). Afterwards cell supernatants have been analyzed every other day for cytokines like IL-1, II-4, II-6, Iba-1, IFN-γ, BDNF, Eotaxin, TNF-alpha, NFκB using Meso Scale Discovery assay platform (MSD, Gaithersburg, Maryland, USA) and NO-level in the cell supernatant has been defined using a Griess-Assay. NO is an important metabolite which can be associated to neurodegenerative disorders because of its pathological conditions, it mainly reflects toxicity. The timeline and sequence of administration as well as the administered concentrations are shown in figure 2.

### B: Experimental design for in vivo mediated activation of adult neurogenesis and the treatment induced by increased levels of TGFβ in rats by the G-CSF/GM-CSF:

Increased CSF (cerebrospinal fluid) TGFβ levels are known to be a hallmark of neurodegenerative processes resulting in reduced neurogenesis. Chronic intracerebroventricular (icv) TGFβ administration via osmotic minipumps serves as a model for the simulation of neurodegenerative processes. The major goal of the following experiments was to investigate whether granulocyte-colony stimulating factor (G-CSF), granulocyte-macrophage colony-stimulating factor (GM-CSF), Lonquex (Lipegfilgrastim) or different combinations are able to prevent, reactivate or compensate the reduced neurogenesis induced by increased levels of circulating TGFβ in a dose-dependent manner.

### Experiment B1: Subcutaneous injections of G-CSF / GM-CSF / G-CSF+GM-CSF / Lonquex / Lonquex + GM-CSF for the prevention of TGFβ-induced reduction of adult neurogenesis

In order to investigate whether G-CSF, GM-CSF, Lonquex or different combinations are able to prevent the reduced neurogenesis induced by increased levels of TGFβ, two- to three-month-old (180 g) female Fisher-344 rats were split into 15 weight-matched groups, a control (VEH, n = 3), a G-CSF_{low} (n = 3), a G-CSF_{high} (n = 3), a GM-CSF_{low} (n = 3), a GM-CSF_{high} (n = 3), a G-CSF_{low}+GM-CSF_{low} (n = 3), a G-CSF_{high}+GM-CSF_{high} (n = 3), a G-CSF_{low}+GM-CSF_{high} (n = 3), a G-CSF_{high}+GM-CSF_{l0w} (n = 3), a Lonquex_{low} (n = 3), a Lonquex _{high} (n = 3), Lonquex_{low}+GM-CSF_{low} (n = 3), a Lonquex_{high}+GM-CSF_{high} (n = 3), a Lonquex_{low}+GM-CSF_{high} (n = 3), and a Lonquex_{high}+GM-CSF_{low} group (n = 3). After a 2-week pretreatment with the respective drug via daily subcutaneous (sc) injections all animals received icv recombinant TGFβ (dissolved in artificial CSF; 1.66 µg/ml) infusions through stainless steel cannulas connected to osmotic minipumps (model 2006, ALZET; Durect Corp., Cupertino, CA) at a flow rate of 0.15 µl/hr. All animals received their respective treatment drug via daily sc injections throughout the entire 6 week (week 3 to week 8) pump period. Every single week blood samples were taken for FACS analysis of monocytes, thrombocytes and eosinophils. In week 8 all rats obtained daily intraperitoneal (ip) injections of 50 mg/kg 5-bromo-2'-deoxyuridine (BrdU) for 7 consecutive days. After killing (at the last day of the pump period, by decapitation following CO₂ anesthesia) brains were rapidly removed, snap-frozen in methylbutane cooled on dry-ice and stored at -80°C for subsequent fluorescence immunodetections. Brains were cut into 40-µm sagittal sections and analysed for proliferation/newborn cells (BrdU), apoptosis (TUNEL), neuronal precursors/immature neurons (doublecortin), mature neurons (NeuN), TGFβR1/2, neural stem cells (nestin), glial cells (GFA) and angiogenesis (VEGF) within the hippocampus and the subventricular zone. As an additional readout parameter for neurogenesis, CSF was taken after decapitation and analysed for doublecortin using Meso Scale Discovery assay platform (MSD, Gaithersburg, Maryland, USA). Following decapitation, trunk blood was collected for immunological analysis using Meso Scale Discovery assay platform (MSD, Gaithersburg, Maryland, USA).

### Preventive use of G-CSF/GM-CSF /Lonquex for TGF-B-induced reduction of adult neurogenesis

sc injection of
G-CSF, GM-CSF, G-CSF+GM-CSF,
Lonquex, Lonquex + GM-CSF
icv infusion of
TGF-β
+
sc injection of
G-CSF, GM-CSF, G-CSF+GM-CSF,
Lonquex, Lonquex + GM-CSF
week 1 | week 2 | week 3 | week 8
pump implantation
BrdU ip injection
tissue collection

| Group | 1 | 2 | 3 | 4 | 5 |
|---|---|---|---|---|---|
| Treatment | saline | G-CSF_{low} | G-CSF_{high} | GM-CSF_{low} | GM-CSF_{high} |
| dose/day/kg for a 200 g rat | 2 ml | 30 µg/kg | 100 µg/kg | 30 µg/kg | 100 µg/kg |

| Group | 6 | 7 | 8 | 9 | 10 |
|---|---|---|---|---|---|
| Treatment | G-CSF_{low} +GM-CSF_{low} | G-CSF_{high} +GM-CSF_{high} | G-CSF_{low} +GM-CSF_{high} | G-CSF_{high} +GM-CSF_{low} | Lonquex_{low} |
| dose/day/kg for a 200 g rat | 30 µg/kg each | 100 µg/kg each | 30 µg/kg 100 µg/kg | 100 µg/kg 30 µg/kg | 30 µg/kg |

| Group | 11 | 12 | 13 | 14 | 15 |
|---|---|---|---|---|---|
| Treatment | Lonquex_{high} | Lonquex_{low} +GM-CSF_{loW} | Lonquex_{high} +GM-CSF_{high} | Lonquex_{low} +GM-CSF_{high} | Lonquex_{high} +GM-CSF_{low} |
| dose/day/kg for a 200 g rat | 100 µg/kg | 30 µg/kg each | 100 µg/kg each | 30 µg/kg 100 µg/kg | 100 µg/kg 30 µg/kg |

| | | | | | |
|---|---|---|---|---|---|
| Volume osmotic minipump: 0.15 µl/hr; 3.6 µl/d TGF-beta: 6 ng/d => 1.66 µg/ml within pump | | | | | |

### Experiment B2: Subcutaneous injection of G-CSF/GM-CSF/G-CSF+GM-CSF/Lonquex/ Lonquex + GM-CSF for the therapeutical treatment/reactivation of TGFβ-induced reduction of adult neurogenesis

In order to investigate whether G-CSF, GM-CSF, Lonquex or different combinations are able to reactivate the reduced neurogenesis induced by increased levels of TGFβ, two- to three-month-old (180 g) female Fisher-344 rats are split into 15 weight-matched groups, a control (VEH, n = 3), a G-CSF_{low} (n = 3), a G-CSF_{high} (n = 3), a GM-CSF_{low} (n = 3), a GM-CSF_{high} (n = 3), a G-CSF_{low}+GM-CSF_{low} (n = 3), a G-CSF_{high}+GM-CSF_{high} (n = 3), a G-CSF_{low}+GM-CSF_{high} (n = 3), a G-CSF_{high}+GM-CSF_{low} (n = 3), a Lonquex _{low} (n = 3), a Lonquex _{high} (n = 3), Lonquex_{low}+GM-CSF_{low} (n = 3), a Lonquex_{high}+GM-CSF_{high} (n = 3), a Lonquex_{low}+GM-CSF_{high} (n = 3), and a Lonquex_{high}+GM-CSF_{low} group (n = 3). All animals received icv recombinant TGFβ (dissolved in artificial CSF; 1.66 µg/ml) infusions through stainless steel cannulas connected to osmotic minipumps (model 2006, ALZET; Durect Corp., Cupertino, CA) at a flow rate of 0.15 µl/hr. After two weeks of a chronic central TGFβ administration, all animals received their respective treatment drug via daily sc injections throughout the remaining 4 week pump period. Every single week blood samples were taken for FACS analysis of monocytes, thrombocytes and eosinophils. In week 6 all rats obtained daily ip injections of 50 mg/kg 5-bromo-2'-deoxyuridine (BrdU) for 7 consecutive days. After killing (at the last day of the pump period, by decapitation following CO₂ anesthesia) brains were rapidly removed, snap-frozen in methylbutane cooled on dry-ice and stored at -80°C for subsequent fluorescence immunodetections. Brains were cut into 40-µm sagittal sections and analysed for proliferation/newborn cells (BrdU), apoptosis (TUNEL), neuronal precursors/immature neurons (doublecortin), mature neurons (NeuN), TGFβR1/2, neural stem cells (nestin), glial cells (GFA) and angiogenesis (VEGF) within the hippocampus and the subventricular zone. As an additional readout parameter for neurogenesis, CSF was taken after decapitation and analysed for doublecortin using Meso Scale Discovery assay platform (MSD, Gaithersburg, Maryland, USA). Following decapitation, trunk blood was collected for immunological analysis using Meso Scale Discovery assay platform (MSD, Gaithersburg, Maryland, USA).

### Therapeutical use of G-CSF/GM-CSF/Lonquex for TGF-β-induced reduction of adult neurogenesis

icv infusion of
TGF-β
icv infusion of
TGF-β
+
sc injection of
G-CSF, GM-CSF, G-CSF+GM-CSF,
Lonquex, Lonquex + GM-CSF
week 1 | week 2 | week 3 | week 4 | week 5 | week 6
pump implantation
BrdU ip injection
tissue collection

| Group | 1 | 2 | 3 | 4 | 5 |
|---|---|---|---|---|---|
| Treatment | saline | G-CSF_{low} | G-CSF_{high} | GM-CSF_{low} | GM-CSF_{high} |
| dose/day/kg for a 200 g rat | 2 ml | 30 µg/kg | 100 µg/kg | 30 µg/kg | 100 µg/kg |

| Group | 6 | 7 | 8 | 9 | 10 |
|---|---|---|---|---|---|
| Treatment | G-CSF_{low} | G-CSF_{high} | G-CSF_{low} | G-CSF_{high} | Lonquex_{low} |
| | +GM-CSF_{low} | +GM-CSF_{high} | +GM-CSF_{high} | +GM-CSF_{low} | |
| dose/day/kg for a 200 g rat | 30 µg/kg each | 100 µg/kg each | 30 µg/kg 100 µg/kg | 100 µg/kg 30 µg/kg | 30 µg/kg |

| Group | 11 | 12 | 13 | 14 | 15 |
|---|---|---|---|---|---|
| Treatment | Lonquex_{high} | Lonquex_{low} +GM-CSF_{loW} | Lonquex_{high} +GM-CSF_{high} | Lonquex_{low} +GM-CSF_{high} | Lonquex_{high} +GM-CSF_{low} |
| dose/day/kg for a 200 g rat | 100 µg/kg | 30 µg/kg each | 100 µg/kg each | 30 µg/kg 100 µg/kg | 100 µg/kg 30 µg/kg |

| | | | | | |
|---|---|---|---|---|---|
| Volume osmotic minipump: 0.15 µl/hr; 3.6 µl/d TGF-beta: 6 ng/d => 1.66 µg/ml within pump | | | | | |

### C: Experimental design for in vivo mediated activation of adult neurogenesis and the treatment of Huntington's disease by the G-CSF/GM-CSF:

It was of further interest whether G-CSF, GM-CSF, Lonquex (Lipegfilgrastim) or different combinations are able to prevent or delay the establishment of a Huntington's disease like phenotype in the R6/2 mouse model and whether they can act as a medication for Huntington's disease.

### Experiment C1: Subcutaneous injection of G-CSF/GM-CSF/G-CSF+GM-CSF/Lonquex/ Lonquex + GM-CSF for the prevention of Huntington's disease in the R6/2 mouse model

In order to investigate whether G-CSF, GM-CSF, Lonquex or different combinations are able prevent or to delay the establishment a of Huntington's disease phenotype, 3-week old R6/2 mice are split into 15 weight-matched groups, a control (VEH, n = 3), a G-CSF_{low} (n = 3), a G-CSF_{high} (n = 3), a GM-CSF_{low} (n = 3), a GM-CSF_{high} (n = 3), a G-CSF_{low}+GM-CSF_{low} (n = 3), a G-CSF_{high}+GM-CSF_{high} (n = 3), a G-CSF_{low}+GM-CSF_{high} (n = 3), a G-CSF_{high}+GM-CSF_{low} (n = 3), a Lonquex _{low} (n = 3), a Lonquex high (n = 3), Lonquex_{low}+GM-CSF_{low} (n = 3), a Lonquex_{high}+GM-CSF_{high} (n = 3), a Lonquex_{low}+GM-CSF_{high} (n = 3), and a Lonquex_{high}+GM-CSF_{low} group (n = 3). One week after arrival, all animals received their respective treatment drug via daily sc injections throughout the following 17 weeks. Motor tests were performed every single week for the first 5 weeks of the experiment. Blood samples were taken every single week for analysis of monocytes, thrombocytes and eosinophils. After killing (end of week 17 of the experiment, by decapitation following CO₂ anesthesia) brains were rapidly removed, snap-frozen in methylbutane cooled on dry-ice and stored at -80°C for subsequent fluorescence immunodetections. Brains were cut into 40-µm sagittal sections and analysed for proliferation/newborn cells (BrdU), apoptosis (TUNEL), neuronal precursors/immature neurons (doublecortin), mature neurons (NeuN), TGFβR1/2, neural stem cells (nestin), glial cells (GFA) and angiogenesis (VEGF) within the hippocampus and the subventricular zone. As an additional readout parameter for neurogenesis, cerebrospinal fluid was taken after decapitation and analysed for doublecortin using Meso Scale Discovery assay platform (MSD, Gaithersburg, Maryland, USA). Following decapitation, trunk blood was collected for immunological analysis using Meso Scale Discovery assay platform (MSD, Gaithersburg, Maryland, USA).

### Preventive use of G-CSF/GM-CSF /Lonquex for Huntington's disease

sc injection of
G-CSF, GM-CSF, G-CSF+GM-CSF, + body weight
Lonquex, Lonquex + GM-CSF
week 1 | week 2 | week 3 | week 4 | week 5 | | | | week 17 |
motor tests
motor tests
motor tests
motor tests
motor tests
BrdU ip injection
tissue collection

| Group | 1 | 2 | 3 | 4 | 5 |
|---|---|---|---|---|---|
| Treatment | saline | G-CSF_{low} | G-CSF_{high} | GM-CSF_{low} | GM-CSF_{high} |
| dose/day/kg for a 25 g mouse | 2 ml | 30 µg/kg | 100 µg/kg | 30 µg/kg | 100 µg/kg |

| Group | 6 | 7 | 8 | 9 | 10 |
|---|---|---|---|---|---|
| Treatment | G-CSF_{low} | G-CSF_{high} | G-CSF_{low} | G-CSF_{high} | Lonquex_{low} |
| | +GM-CSF_{low} | +GM-CSF_{high} | +GM-CSF_{high} | +GM-CSF_{low} | |
| dose/day/kg for a 25 g mouse | 30 µg/kg each | 100 µg/kg each | 30 µg/kg 100 µg/kg | 100 µg/kg 30 µg/kg | 30 µg/kg |

| Group | 11 | 12 | 13 | 14 | 15 |
|---|---|---|---|---|---|
| Treatment | Lonquex_{high} | Lonquex_{low} | Lonquex_{high} | Lonquex_{low} | Lonquex_{high} |
| | | +GM-CSF_{loW} | +GM-CSF_{high} | +GM-CSF_{high} | +GM-CSF_{low} |
| dose/day/kg for a 25 g mouse | 100 µg/kg | 30 µg/kg each | 100 µg/kg each | 30µg/kg 100 µg/kg | 100 µg/kg 30 µg/kg |

### Experiment C2: Subcutaneous injection of G-CSF/GM-CSF/G-CSF+GM-CSF/Lonquex/ Lonquex + GM-CSF for the therapeutical treatment of Huntington's disease in the R6/2 mouse model

In order to investigate whether G-CSF, GM-CSF, Lonquex or different combinations are able to act as a potential medication for the treatment of Huntington's disease, 3-week old R6/2 mice are split into 15 weight-matched groups, a control (VEH, n = 3), a G-CSF_{low} (n = 3), a G-CSF_{high} (n = 3), a GM-CSF_{low} (n = 3), a GM-CSF_{high} (n = 3), a G-CSF_{low}+GM-CSF_{low} (n = 3), a G-CSF_{high}+GM-CSF_{high} (n = 3), a G-CSF_{low}+GM-CSF_{high} (n = 3), a G-CSF_{high}+GM-CSF_{low} (n = 3), a Lonquex _{low} (n = 3), a Lonquex_{high} (n = 3), Lonquex_{low}+GM-CSF_{low} (n = 3), a Lonquex_{high}+GM-CSF_{high} (n = 3), a Lonquex_{low}+GM-CSF_{high} (n = 3), and a Lonquex_{high}+GM-CSF_{low} group (n = 3). After developing the first Huntington's disease like symptoms (after 3 weeks), all animals received their respective treatment drug via daily subcutaneous (sc) injections throughout the following 14 weeks. Motor tests were performed every single week for the first 5 weeks of the experiment. Blood samples were taken every single week for the analysis of monocytes, thrombocytes and eosinophils. After killing (end of week 17 of the experiment, by decapitation following CO₂ anesthesia) brains were rapidly removed, snap-frozen in methylbutane cooled on dry-ice and stored at-80°C for subsequent fluorescence immunodetections. Brains were cut into 40-µm sagittal sections and analysed for proliferation/newborn cells (BrdU), apoptosis (TUNEL), neuronal precursors/immature neurons (doublecortin), mature neurons (NeuN), TGFβR1/2, neural stem cells (nestin), glial cells (GFA) and angiogenesis (VEGF) within the hippocampus and the subventricular zone. As an additional readout parameter for neurogenesis, CSF was taken after decapitation and analyzed for doublecortin using Meso Scale Discovery assay platform (MSD, Gaithersburg, Maryland, USA). Following decapitation, trunk blood was collected for immunological analysis using Meso Scale Discovery assay platform (MSD, Gaithersburg, Maryland, USA).

### Therapeutical use of G-CSF/GM-CSF /Lonquex for Huntington's disease

sc injection of
G-CSF, GM-CSF, G-CSF+GM-CSF, + body weight
Lonquex, Lonquex + GM-CSF
week 1 | week 2 | week 3 | week 4 | week 5 | | | | week 17 |
motor tests
motor tests
motor tests
motor tests
motor tests
BrdU ip injection
tissue collection

| Group | 1 | 2 | 3 | 4 | 5 |
|---|---|---|---|---|---|
| Treatment | saline | G-CSF_{low} | G-CSF_{high} | GM-CSF_{low} | GM-CSF_{high} |
| dose/day/kg for a 25 g mouse | 2 ml | 30 µg/kg | 100 µg/kg | 30 µg/kg | 100 µg/kg |

| Group | 6 | 7 | 8 | 9 | 10 |
|---|---|---|---|---|---|
| Treatment | G-CSF_{low} +GM-CSF_{low} | G-CSF_{high} +GM-CSF_{high} | G-CSF_{low} +GM-CSF_{high} | G-CSF_{high} +GM-CSF_{low} | Lonquex_{low} |
| dose/day/kg for a 25 q mouse | 30 µg/kg each | 100 µg/kg each | 30 µg/kg 100 µg/kg | 100 µg/kg 30 µg/kg | 30 µg/kg |

| Group | 11 | 12 | 13 | 14 | 15 |
|---|---|---|---|---|---|
| Treatment | Lonquex_{high} | Lonquex_{low} +GM-CSF_{loW} | Lonquex_{high} +GM-CSF_{high} | Lonquex_{low} +GM-CSF_{high} | Lonquex_{high} +GM-CSF_{low} |
| dose/day/kg for a 25 g mouse | 100 µg/kg | 30 µg/kg each | 100 µg/kg each | 30 µg/kg 100 µg/kg | 100 µg/kg 30 µg/kg |

### Results

The examples demonstrate the advantageous effect of the administration of the combination of G-CSF and GM-CSF or the pegylated derivatives thereof over the administration of only G-CSF or only GM-CSF in the treatment or prophylaxis of neurological diseases.

### Description of Figures

- Fig. 1:: Shows the timeline and sequence of administration as well as the administered concentrations used in Example A1 and Example A2.
- Fig. 2:: Shows the timeline and sequence of administration as well as the administered concentrations used in Example A3.

## Claims

1. A combination of hematopoietic growth factors for use in the treatment or prophylaxis of neurological diseases selected from diseases associated with ischemia or hypoxia mechanisms, neurodegenerative diseases, neurological and psychiatric disorders associated with neural cell death, wherein a first hematopoietic growth factor is selected from the group consisting of G-CSF, functional active variants of G-CSF having at least 90 % sequence identity to G-CSF and conjugated G-CSF, and a second hematopoietic growth factor is selected from the group consisting of GM-CSF, functional active variants of GM-CSF having at least 90 % sequence identity to GM-CSF and conjugated GM-CSF.

2. The combination of hematopoietic growth factors according to claim 1, wherein neurodegenerative disease is selected from the group consisting of amyotrophic lateral sclerosis, Parkinson's disease, Parkinson-like diseases, Huntington's disease, spinal muscular-atrophy, progressive supranuclear palsy, Wilson's disease, multi-system atrophy, striatonigral degeneration, Shy-Drager syndrome, Alzheimer's disease, spinocerebellar ataxia, glaucoma, Pick's disease, Lewy-body disease, Hallervorden-Spatz disease, torsion dystonia, hereditary sensorimotor neuropathies, Gerstmann-Straussler-Schanker disease, Creutzfeld-Jakob-disease, Machado-Joseph disease, Friedreich ataxia, non-Friedreich ataxias, Gilles de la Tourette syndrome, familial tremors, olivopontocerebellar degenerations, paraneoplasticcerebral syndromes, hereditary spastic paraplegias, hereditary optic neuropathy, degenerative retinal diseases, retinitis pigmentosa, dry and humid macular degeneration, Stargardt disease, and Keams-Sayre syndrome.

3. The combination of hematopoietic growth factors according to claim 1 or 2, wherein the conjugated G-CSF consists of a non-immunogenic hydrophilic polymer covalently linked to G-CSF or wherein the conjugated GM-CSF consists of a non-immunogenic hydrophilic polymer covalently linked to GM-CSF.

4. The combination of hematopoietic growth factors according to claim 3, wherein the non-immunogenic hydrophilic polymer is selected from the group consisting of polyethylene glycols, polyoxypropylenes, polyoxyethylene-polyoxypropylene block copolymers, polyvinylpyrrolidones, polyacyloylmorpholines, polysaccharides, and aminocarbamyl polyethylene glycols.

5. The combination of hematopoietic growth factors according to claim 3 or 4, wherein the non-immunogenic hydrophilic polymer is selected from linear or branched monomethoxy-polyethylene glycol amine and aminocarbamyl polyethylene glycol having a molecular weight from 5kDa to 40kDa.

6. The combination of hematopoietic growth factors according to any one of claims 1 - 5, wherein the conjugated G-CSF is a site-specific G-CSF mono-conjugate and wherein the site-specific G-CSF mono-conjugate consists of a non-immunogenic hydrophilic polymer covalently linked to a glutamine residue in the position 134 of the native human 174-aminoacid G-CSF polypeptide chain sequence or 135 of the 175-aminoacid Met-G-CSF polypeptide chain sequence.

7. The combination of hematopoietic growth factors according to any one of claims 1 - 6, wherein the prophylaxis involves determination of the prevalence or risk to obtain a neurological disease selected from diseases associated with ischemia or hypoxia mechanisms, neurodegenerative diseases, neurological and psychiatric disorders associated with neural cell death by detecting genetic mutations of the person indicating the prevalence or risk to obtain the neurological disease.

8. The combination of hematopoietic growth factors according to any one of claims 1 - 7, wherein the determination of the prevalence or risk to obtain a neurological disease is based on the detection of diagnostic biomarkers selected from single nucleotide polymorphisms in FLJ10986, PLXDC2, FOXA2, CGNL1, NOX4, C6orf170, IQGAP2, CGNL1, Splicest EST, LOXHD1, ACCN1, TMCC2, FATE1, PTPRT, MAGI2, TACR2, DBF4B, ADAMTS20, NFIA, WNT9A, GARNL4, IL18RAP, TUFT1, TIAM2, DSC3, PARP8, DGKB, NELL1, ALK, ZNFN1A2, NGNL6975, BM924006, PRDM1, SLCO3A1, DA629334, IL2RB, PCF11, SOX21, GNG7, EIF5A, FBXO15, wherein the neurological disease is selected from ALS and/or, Alzheimer's disease and/or, Parkinson's disease and/or, Parkinson's dementia.

9. The combination of hematopoietic growth factors according to any one of claims 1 - 8 comprising the first hematopoietic growth factor selected from the group consisting of G-CSF, functional active variants of G-CSF having at least 90 % sequence identity to G-CSF and conjugated G-CSF, and the second hematopoietic growth factor selected from the group consisting of GM-CSF, functional active variants of GM-CSF having at least 90 % sequence identity to GM-CSF and conjugated GM-CSF in a single dose in the range of 0,5µg/kg to 5 µg/kg per day.

10. The combination of hematopoietic growth factors according to any one of claims 1 - 9 for use in the treatment or prophylaxis by intrathecal and/or intracerebroventricular administration.

11. The combination of hematopoietic growth factors according to any one of claims 1 - 10 comprising a viral vector or a liposome as vehicle for the combination.

12. A pharmaceutical composition containing a combination of two hematopoietic growth factors in a ratio of 1 to 1 of the first one to the second one and the first one is selected from the group consisting of G-CSF, functional active variants of G-CSF having at least 90 % sequence identity to G-CSF and conjugated G-CSF, and the second one is selected from the group consisting of GM-CSF, functional active variants of GM-CSF having at least 90 % sequence identity to GM-CSF and conjugated GM-CSF together with a pharmaceutically acceptable solvent, diluent, carrier and/or adjuvant.

13. A pharmaceutical composition according to claim 12, wherein the combination of two hematopoietic growth factors is in a ratio of 2 to 1 of the first one to the second one.

14. A pharmaceutical composition according to claims 12 or 13, wherein the combination of two hematopoietic growth factors is in a ratio of 1 to 2 of the first one to the second one.

15. The pharmaceutical composition according to one of claims 12 - 14 further comprising a viral vector or a liposome as vehicle for the combination.


SEQUENCE LISTING

<110>  NeuroVision Pharma GmbH

<120>  Pharmaceutical composition consisting of a combination
       of G-CSF with GM-CSF

<130>  REG-P03597EP

<160>  3

<170>  PatentIn version 3.5

<210>  1
<211>  174
<212>  PRT
<213>  Homo sapiens

<400>  1

Thr Pro Leu Gly Pro Ala Ser Ser Leu Pro Gln Ser Phe Leu Leu Lys
1               5                   10                  15

Cys Leu Glu Gln Val Arg Lys Ile Gln Gly Asp Gly Ala Ala Leu Gln
            20                  25                  30

Glu Lys Leu Cys Ala Thr Tyr Lys Leu Cys His Pro Glu Glu Leu Val
        35                  40                  45

Leu Leu Gly His Ser Leu Gly Ile Pro Trp Ala Pro Leu Ser Ser Cys
    50                  55                  60

Pro Ser Gln Ala Leu Gln Leu Ala Gly Cys Leu Ser Gln Leu His Ser
65                  70                  75                  80

Gly Leu Phe Leu Tyr Gln Gly Leu Leu Gln Ala Leu Glu Gly Ile Ser
                85                  90                  95

Pro Glu Leu Gly Pro Thr Leu Asp Thr Leu Gln Leu Asp Val Ala Asp
            100                 105                 110

Phe Ala Thr Thr Ile Trp Gln Gln Met Glu Glu Leu Gly Met Ala Pro
        115                 120                 125

Ala Leu Gln Pro Thr Gln Gly Ala Met Pro Ala Phe Ala Ser Ala Phe
    130                 135                 140

Gln Arg Arg Ala Gly Gly Val Leu Val Ala Ser His Leu Gln Ser Phe
145                 150                 155                 160

Leu Glu Val Ser Tyr Arg Val Leu Arg His Leu Ala Gln Pro
                165                 170

<210>  2
<211>  175
<212>  PRT
<213>  Homo sapiens

<400>  2

Met Thr Pro Leu Gly Pro Ala Ser Ser Leu Pro Gln Ser Phe Leu Leu
1               5                   10                  15

Lys Cys Leu Glu Gln Val Arg Lys Ile Gln Gly Asp Gly Ala Ala Leu
            20                  25                  30

Gln Glu Lys Leu Cys Ala Thr Tyr Lys Leu Cys His Pro Glu Glu Leu
        35                  40                  45

Val Leu Leu Gly His Ser Leu Gly Ile Pro Trp Ala Pro Leu Ser Ser
    50                  55                  60

Cys Pro Ser Gln Ala Leu Gln Leu Ala Gly Cys Leu Ser Gln Leu His
65                  70                  75                  80

Ser Gly Leu Phe Leu Tyr Gln Gly Leu Leu Gln Ala Leu Glu Gly Ile
                85                  90                  95

Ser Pro Glu Leu Gly Pro Thr Leu Asp Thr Leu Gln Leu Asp Val Ala
            100                 105                 110

Asp Phe Ala Thr Thr Ile Trp Gln Gln Met Glu Glu Leu Gly Met Ala
        115                 120                 125

Pro Ala Leu Gln Pro Thr Gln Gly Ala Met Pro Ala Phe Ala Ser Ala
    130                 135                 140

Phe Gln Arg Arg Ala Gly Gly Val Leu Val Ala Ser His Leu Gln Ser
145                 150                 155                 160

Phe Leu Glu Val Ser Tyr Arg Val Leu Arg His Leu Ala Gln Pro
                165                 170                 175


<210>  3
<211>  144
<212>  PRT
<213>  Homo sapiens

<400>  3

Met Trp Leu Gln Ser Leu Leu Leu Leu Gly Thr Val Ala Cys Ser Ile
1               5                   10                  15

Ser Ala Pro Ala Arg Ser Pro Ser Pro Ser Thr Gln Pro Trp Glu His
            20                  25                  30

Val Asn Ala Ile Gln Glu Ala Arg Arg Leu Leu Asn Leu Ser Arg Asp
        35                  40                  45

Thr Ala Ala Glu Met Asn Glu Thr Val Glu Val Ile Ser Glu Met Phe
    50                  55                  60

Asp Leu Gln Glu Pro Thr Cys Leu Gln Thr Arg Leu Glu Leu Tyr Lys
65                  70                  75                  80

Gln Gly Leu Arg Gly Ser Leu Thr Lys Leu Lys Gly Pro Leu Thr Met
                85                  90                  95

Met Ala Ser His Tyr Lys Gln His Cys Pro Pro Thr Pro Glu Thr Ser
            100                 105                 110

Cys Ala Thr Gln Ile Ile Thr Phe Glu Ser Phe Lys Glu Asn Leu Lys
        115                 120                 125

Asp Phe Leu Leu Val Ile Pro Phe Asp Cys Trp Glu Pro Val Gln Glu
    130                 135                 140